# EUROPEAN PATENT APPLICATION

(11) **EP 4 537 862 A1**
(43) Date of publication of application: **16.04.2025**
(21) Application number: 23383047.0
(22) Date of filing: 11.10.2023
(51) Int. Cl.: A61L 27/48, A61L 27/52, G02B 1/04

(54) **SILK FIBROIN OPHTHALMIC MATERIAL FOR CORNEAL INLAY AND OTHER OPHTHALMIC APPLICATIONS**

(71) Applicant: Consejo Superior de Investigaciones Cientificas, 28006 Madrid (ES)
(72) Inventor: GUTIERREZ CONTRERAS, Rocio, 28006 Madrid (ES); FERNANDEZ GUTIERREZ, Mar, 28006 Madrid (ES); DE LA HOZ DURAN, Andres, 28006 Madrid (ES); MARCOS CELESTINO, Susana, 28006 Madrid (ES)
(74) Representative: Elion IP, S.L.

(57) **Abstract**

The present disclosure relates to:
- an ophthalmic material obtainable by polymerizing a first monomer and a second monomer in the presence of silk fibroin (SF) wherein the first monomer and the second monomer are different and are independently selected from a methacrylate, an acrylate, an acrylamide, a siloxane, a carbamate, a glycol, a vinyl and an allyl, and wherein the weight ratio of the first monomer to the second monomer is greater than 1:1;
- a hydrogel comprising the ophthalmic material and water, in particular comprising 10 to 40 wt.% of water based on the total weight of the hydrogel;
- an ophthalmic lens or implant comprising the ophthalmic material or the hydrogel; and
- a method for preparing the ophthalmic material.

## Description

### TECHNICAL FIELD

The present disclosure generally relates to the field of ophthalmology. More specifically, the present disclosure relates to a hydrogel for ophthalmic applications.

### BACKGROUND

A large number of synthetic polymers (silicone, acrylics, both hydrophilic and hydrophobic) have been developed for use in ophthalmic lenses and/or implants to perform corrections, e.g., refractive corrections, to the eye to treat, e.g., myopia, hyperopia, astigmatism and/or presbyopia. They come in contact with the eye and in several cases, these are implanted in the eye permanently, and therefore they need to be biocompatible and have a long-term stability in the eye.

Some refractive corrections involve eliminating tissue. For example, in Laser-assisted in situ keratomileusis (LASIK), Photorefractive keratectomy (PRK or SMILE surgery, corneal tissue is ablated or a corneal lenticule is extracted to reshape the cornea and change power. In refractive lens exchange, the transparent natural crystalline lens is replaced by an intraocular lens, for refractive purposes or a correction for presbyopia (with multifocal or extended depth of focus lenses). Subtractive surgery comes at some expense, such as corneal thinning, risk of ectasia or elimination of a functional crystalline lens.

In recent years, refractive procedures have started focusing on additive surgery where tissue is added rather than eliminated. A prominent example of additive surgery are corneal inlays, intrastromal implants (placed the stroma: the thicker middle layer in the cornea), that have been introduced with different degrees of success.

One example of commercially available corneal inlay (e.g., Raindrop^{®}) operates by cornea reshaping, and is claimed to be made of a highly biocompatible proprietary material but needs to be made thin, and very small in diameter, and implanted deep in the cornea. Another example, the Kamra^{®} is a corneal inlay with holes and a small aperture aimed at extending depth-of-focus. Some other commercial inlays (e.g., Flexivue Microlens^{®}) are made out of hydrophilic acrylic material, are also small (3 mm, 20 µm thickness) and have been described to need a hole in the center to allow nutrient flow. Actually, cases of corneal haze have been reported for some such commercial corneal inlays which have limited their use and even prompted FDA recalls.

Biocompatibility and permeability appear to be key factors to the viability of corneal implants. For example, although poly(methyl methacrylate (PMMA) is not toxic in the cornea (i.e. intrastromal ring segments, which are implanted peripherally are generally well tolerated), central PMMA-made corneal inlays have been shown to cause necrosis and implant extrusion, due to the lack of permeability.

It is generally accepted that materials of natural origin will show a higher degree of affinity to the eye's environment, although the solutions proposed to date present several caveats. Solidified collagen or collagen-like corneal inlays were proposed but lack sufficient mechanical strength and stability, resulting in lenticule remodeling and abnormalities of the re-grown corneal epithelium.

Another example are corneal onlays, which differ from corneal inlays in where they are implanted within the cornea, and are placed near the surface of the eye, directly under the thin outer layer of the cornea called the epithelium. Corneal onlays made out of porous perfluoropolyether coated with collagen have demonstrated support for stable stratified squamous epithelium growth when implanted under a superficial stromal flap in a cat model, but were never pursued to demonstrate corneal reshaping.

More recent approaches have looked into corneal inlays derived from biological corneal tissue. Bio-compatibility has been shown in biological corneal inlays for presbyopia, derived from small incision corneal lenticule extraction in a non-human primate study, but only in autogenic or decellularized xenogeneic implantation. Xenia^{®} are intrastromal ring segments made out of decellularized porcine collagen, although the heterogeneity and possibly the lack of biomechanical suitability has been recognized and tried to overcome by cross-linking. Finally, the use of corneal lenticules cut out from donor corneas for corneal inlays/onlays has been proposed commercially by Allotex, Inc, but it is subject to cryo-preservation, tissue access and allogeneic material associated risks.

Another approach has been combining synthetic and biological materials. For instance, the market standard for phakic lens (ICLs, by Staar Surgical Optics) are made of collamer material, with 60% of polyhydroxy ethyl methacrylate (pHEMA), water (36%), benzophenone (3.8%), and 0.2% porcine collagen. The presence of collagen is claimed to have a positive effect in the hydrophilicity and exchange of gas and nutrients in the anterior chamber. Phakic lenses are intraocular lenses that are implanted in the anterior segment of the eye, generally between the cornea and the natural crystalline lens. They are generally aimed at correcting myopia while preserving the natural lens, still accommodating in young eyes, or more recently even presbyopia, preserving a still transparent lens in presbyopic but not cataract patients. Given the high proximity of other ocular structures, which may be posed at risk, light and highly biocompatible materials are required.

There is a need for additional materials which fill the gap between synthetic polymers and allogeneic donor tissue for ophthalmic lenses and/or implants such as corneal implants, phakic intraocular lenses, aphakic intraocular lenses, corneal onlays, intracorneal ring segments, and contact lenses.

### SUMMARY

It has now been found that an ophthalmic material obtainable by polymerizing a first monomer and a second monomer in the presence of silk fibroin (SF), is well suited for ophthalmic lenses and/or implants and in particular for corneal inlay application.

Silk fibroin, a protein produced by the silkworm, is a natural compound and has been found to enhance the properties of the synthetic polymers for eye implantation. It is transparent, biocompatible, and affordable. Silk fibroin is an FDA-approved biomaterial and is being commercialized in the eyecare market in the format of eye drops and silk sutures and proposed in ophthalmology as scaffold for regenerative corneas. It has now been found that when a first monomer and a second monomer are polymerized in the presence of silk fibroin (SF), the mechanical properties of the resulting polymerized silk-based material are well suited for ophthalmic applications, such as ophthalmic lenses and/or implants and in particular for corneal inlay application. The resulting ophthalmic material has now been proved to be bio-compatible and stable.

In particular, the present disclosure relates to:
- an ophthalmic material obtainable by polymerizing a first monomer and a second monomer in the presence of silk fibroin (SF) wherein the first monomer and the second monomer are different and are independently selected from a methacrylate, an acrylate, an acrylamide, a siloxane, a carbamate, a glycol, a vinyl and an allyl, and wherein the weight ratio of the first monomer to the second monomer is greater than 1:1;
- a hydrogel comprising the ophthalmic material and water, in particular comprising 10 to 40 wt.% of water based on the total weight of the hydrogel;
- an ophthalmic lens or implant comprising the ophthalmic material or the hydrogel; and
- a method for preparing the ophthalmic material.

Further aspects and embodiments of the present disclosure are discussed in detail below.

The different aspects and embodiments of the present disclosure defined in the foregoing and the below can be combined with one another, as long as they are compatible with each other. Additional advantages and features will become apparent from the detailed description that follows and will be particularly pointed out in the appended claims.

### DETAILED DESCRIPTION

The following description is not to be taken in a limiting sense but is given solely for the purpose of describing the broad principles of the disclosure. Embodiments of the ophthalmic material, ophthalmic implant or lenses and methods of the present disclosure will be described by way of example.

As indicated above, the disclosure relates to an ophthalmic material obtainable by polymerizing a first monomer and a second monomer in the presence of silk fibroin (also referred to as SF in the present text). Silk fibroin is typically obtained from silk-cocoon in a known fashion.

An ophthalmic material as described herein is understood as material that is suitable for ophthalmic applications, such as a hydrogel. An ophthalmic material as described herein is substantially free of water, e.g., has less than 0.5 wt.% of water. In some embodiments, the ophthalmic material, e.g., when comprising hydrophilic monomers, it may be hydrated upon addition of water to form a hydrogel. Accordingly, the ophthalmic material may also be referred herein as to a dry ophthalmic material o dry hydrogel and the hydrogel obtained may also be referred to as the hydrated ophthalmic material.

As indicated above and as described in more detail below, the ophthalmic material is well suited for ophthalmic lenses and/or implants. The ophthalmic material as such may be used for the formation of said ophthalmic lenses and/or implants or may be hydrated to form the hydrogel to form said ophthalmic lenses and/or implants.

The first and second monomers are monomers suitable for polymerization and may be selected from monomers typically used in ophthalmic applications.

The first monomer and the second monomer are different from each other. As a mode of example, the first and second monomer are independently selected from a methacrylate, an acrylate, an acrylamide, a siloxane, a carbamate, a glycol, a dialdehyde, a vinyl and an allyl.

In several embodiments the methacrylate may be selected from hydroxy ethyl methacrylate (HEMA), ethylene glycol dimethacrylate (EGDMA), methacrylic acid (MAA), methyl methacrylate (MMA), oligo (ethylene glycol) methyl ether methacrylate (OEGMA), glycerol methacrylate (GMA), isobutyl methacrylate (IBMA), allyl methacrylate (AMA), 3-[tris(trimethylsiloxy)silyl] propyl methacrylate (TRIS), polypropylene glycol dimethylacrylate (PPGDMA) and methacryloyl oxyethyl phosphorylcholine (MPC); more preferably may be selected from HEMA and EGDMA; and yet more preferably the first monomer may be HEMA and/or the second monomer may be EGDMA.

As a mode of example an acrylate may be ethylene glycol phenyl ether acrylate (EGPEA); an acrylamide may be selected from N,N-dimethylacrylamide (DMA), diacetone acrylamide (DAA) and methylene-bis-acrylamide (MBA); a siloxane may be dimethylsiloxane (DMS), a carbamate may be tris(trimethylsioxy)silyl) propyl vinyl carbamate (TPVC), a glycol may be selected from diethylene glycol (DEG) and polyethylene glycol dialdehyde (PEG-DA), a vinyl may be N-vinyl pyrrolidone (N-VP) and an allyl may be diallyl maleate (DA).

The first monomer may be preferably selected from a methacrylate such as HEMA, EGDMA, MAA, MMA, OEGMA, GMA, IBMA, AMA, TRIS; an acrylate such EGPEA; a methacrylamide DMA and DAA; a siloxane such as DMS; a carbamate such as TPVC; and a glycol such as DEG. Preferably the first monomer may be HEMA.

The second monomer may be preferably selected from a methacrylate such as EGDMA and PPGDMA; an acrylamide such as MBA; a glycol such as PEG-DA; a vinyl such as N-VP; and an allyl such as DA. Preferably the first monomer may be EGDMA.

In several particular embodiments the first and second monomer may be selected from hydrophilic monomers, in particular they may be selected from HEMA, EGDMA, EGPEA, DEG, and MPC, and more in particular from HEMA and EGDMA. In a preferred embodiment the first monomer is HEMA and/or the second monomer is EGDMA. In yet a preferred embodiment the first monomer is HEMA and the second monomer is EGDMA. The use of such monomers may advantageously provide an ophthalmic material suitable for forming a hydrogel. Hydrogels also find applications in ophthalmic lenses or implants.

An ophthalmic material as described herein may typically have a weight proportion of the combination of the first and second monomers with respect to SF of 10-40 wt.% to 60-90 wt.%, and more in particular, 20-25 wt.% to 75-80 wt.% based on the total weight of first monomer, second monomer and SF.

An ophthalmic material as described herein may typically have a weight ratio of the first monomer to the second monomer is from 2:1 to 40:1, in particular from 3:1 to 35:1, more in particular from 5:1 to 30:1, yet more in particular from 7:1 to 25:1.

As indicated above, in several embodiments, e.g., when the first and second monomers of the ophthalmic material, are selected from hydrophilic monomers, upon addition of water the ophthalmic material may form a hydrogel.

Accordingly, the instant disclosure further relates to a hydrogel comprising the ophthalmic material as described herein and water. In several embodiments the hydrogel may comprise 10 to 40 wt.% of water, in particular from 12 to 30 wt.% of water, based on the total weight of the hydrogel.

A hydrogel as described herein has been found to have properties particularly suitable for ophthalmic applications and in particular when used as ophthalmic lens or implant. Further their properties may be modulated to adapt for particular applications.

A hydrogel as described herein typically has good transparency, which is indicated by high transmittance values. For instance, the transmittance of the hydrogel may be of over 90%, in particular of over 98%, averaged from, e.g., five measurements following a standard transmittance method using a UV/Vis spectrophotometer. The hydrogel samples are placed in a cuvette with water. The absorbance or transmission of light at the different wavelengths across the visible range, 400-800 nm, was measured with the spectrophotometer, which provides data on the intensity of transmitted or absorbed light. The transmittance measurement over the five samples was averaged within the 400-800 nm visible range. Transmittance of the hydrogel was measured to be of 98%, which is similar to that of intraocular lenses. Having high transparency is an important property that makes the ophthalmic material particularly suited for ophthalmic applications such as an ophthalmic lens and/or implant.

A hydrogel as described herein typically has a good biocompatibility. The biocompatibility may be assessed in terms of cytotoxicity and off cell adhesion, growth, and proliferation.

On one hand, cells in contact with a hydrogel as described herein may typically have cell viability above 80% as determined by the MTT cytotoxicity test according to ISO 10993, in particular viabilities of 100% are typically obtained for a hydrogel as described herein. This ISO relates to the biological evaluation of medical devices, and in particular provides tests for measuring in vitro cytotoxicity. Briefly the MTT test is based on measuring the viability of cells via metabolic activity. In particular, yellow water-soluble MTT (3-(4,5-dimethylthiazol-2-yl)-2,5-diphenyltetrazoliumbromid) is metabolically reduced in viable cells to a blue-violet insoluble formazan. The number of viable cells correlates to the color intensity determined by photometric measurements after dissolving the formazan in DMSO. The viability may be expressed as a percentage of viable cells after being put in contact with the material being assessed with respect to the total viable cells cultured under the same conditions which have not been in contact with the material.

As a mode of example, for assessing the viability of the hydrated ophthalmic material for optical applications, cells derived from human cornea may be used in the MTT test, as a mode of example the MTT test may be performed using Human corneal fibroblasts (HCFs) or human cornea epithelial cells (HCE) immortalized by modification with the plasmid SV-40. Generally speaking, a material may be considered cytotoxic if its cellular viability does not exceed 70%. Hydrogels as described herein, displaying viabilities over 80 % and typically of 100% are found to be highly biocompatible.

On the other, a hydrogel as described herein has also been found to decrease cell proliferation, indicating that the ophthalmic material is non-toxic, and prevent conversion of fibroblasts into myoblasts, which is a marker of possible opacification, when compared to a control, e.g., plastic material for cell culture. In particular, cell adhesion, growth, and proliferation assays may also be performed with HCF, with direct seeding on the hydrogel and using phase contrast optical microscopy and Presto blue assay (PB), a redox indicator that changes color in response to the chemical reduction of the culture medium that occurs as a consequence of cell growth and proliferation. The assay may be performed with several steps of reagent removal, replacement with fresh medium and monitoring of cell proliferation, e.g., at 1, 5 and 7 days.

A hydrogel as described herein typically has a good stability. For instance, the stability of the hydrogel may be of over 2 years in, e.g., a phosphate buffered saline (PBS) solution at 37° C, which are similar conditions to the physiological conditions found in the eye, and of over 12 months in, e.g., 0.05 U/mL protease XIV solution, which are conditions that are more aggressive to those found in the eye. This makes the ophthalmic material particularly suited for ophthalmic applications such as an ophthalmic lens and/or implant, which require to withstand optical conditions for a length of time, from a few days to several years.

In several embodiments a hydrogel as described herein may have a Young's modulus from 20 to 70 MPa, in particular from 30 to 60 MPa, as measured by uniaxial tensile testing. Providing such Young's modulus values can advantageously contribute to mechanical strength and stability, since the hydrogel has higher stiffness than the cornea's stiffness, and is therefore less deformable.

In several embodiments a hydrogel as described herein may have a glucose permeability, which is indicated by diffusion values of at least 10⁻⁶ cm²/s, and in particular, of 10⁻⁷ cm²/s to 10⁻⁸ cm²/s, as measured by a custom-made system. The custom-made system used for the permeability measurements is made up of two chambers, which are screwed to a central piece, where the hydrogel is placed. The hydrogel creates a barrier between both chambers. One chamber is filled with 10 mg/mL glucose solution in PBS, and the other chamber is filled with PBS. The system is placed horizontally on an orbital shaker for 24 hours. After this time, the amount of glucose that has diffused through the hydrogel from the glucose chamber to the PBS chamber is measured with a glucose assay kit. The glucose diffusion coefficient is calculated using the following equation: Q = (D·C1 ·t)/L, where Q is the amount of glucose that passes through the material in time t per unit area; D is the diffusion coefficient; C1 is the initial glucose concentration in the glucose chamber and L is the thickness of the hydrogel samples. Providing such permeability property makes the ophthalmic material particularly suited for ophthalmic applications such as an ophthalmic lens and/or implant.

As indicated above, the ophthalmic material is particularly suited for ophthalmic applications and for use as ophthalmic lens and/or implant. Accordingly, the present disclosure also relates to and ophthalmic lens and/or implant comprising an ophthalmic material or the hydrogel as described herein. It should be noted that some ophthalmic lenses can be implanted in the eye, and are also within the term ophthalmic implant.

In several embodiments an ophthalmic lens or implant may be a selected from a corneal inlay, a phakic lens, an intraocular lens, an aphakic intraocular lens, a corneal onlay, an intracorneal ring segment (for keratoconus or for corneal applanation) and a contact lens. The ophthalmic lens or implant may preferably be a corneal inlay.

These ophthalmic lenses or implants are known in the art and the dimensions and parameters typically applicable to such ophthalmic lenses or implants are also applicable to an ophthalmic lens or implant comprising the ophthalmic material or the hydrogel as described herein.

As mentioned above and discussed in more detail below the ophthalmic lens or implant may be formed from the ophthalmic material or the hydrogel. If formed from the ophthalmic material the ophthalmic lens or implant is hydrated prior to use a lens or implant as also detailed below. Thus, relevant properties to the optical application of the ophthalmic lens or implant are therefore of the hydrated ophthalmic lens or implant as it will be operational in its final application. In the present description they hydrated ophthalmic lens or implant may be used to refer indistinctively to the ophthalmic lens or implant obtained from the ophthalmic material and which is subsequently hydrated or from ophthalmic lens or implant directly obtained from a hydrogel as described herein, and they will both also comprise the hydrogel formed upon hydration of the ophthalmic material.

In several embodiments, an ophthalmic lens or implant comprises the ophthalmic material and is a corneal inlay.

In several embodiments, the ophthalmic lens or implant is a corneal inlay and comprising a hydrogel as described herein may have an essentially circular shape and a diameter between 2-2.5 mm and/or may have a central thickness between 15-30 µm.

In several embodiments, the ophthalmic lens or implant is a corneal inlay and comprises a hydrogel as described herein, and is shaped as a 3d-form having an anterior surface and a posterior surface, both surfaces being essentially curve and having curvature radii of between 6 mm to 10 mmm, and more preferably, of between 6 mm to 8 mm. In the context of the present disclosure, the terms anterior or outermost, and posterior or innermost, can be indistinctly used, and refer to the position of use of the ophthalmic lens and/or implant.

The ophthalmic material obtained as indicated in the foregoing is particularly suitable for its use in corneal inlays, in order to reshape the cornea for refractive or presbyopia treatments. From the ophthalmic material, such a corneal inlay can be obtained by moulding, lathing or machining with lasers, as described, for instance, in Applegate, M.B., et al., "A simple model of multiphoton micromachining in silk hydrogels". Applied Physics Letters, 2016. 108(24).

In certain embodiments, corneal inlays are designed based on measured mechanical properties of the hydrogel, which produces a three-dimensional modification in the central corneal curvature. The corneal inlay can be shaped as a positive meniscus. The corneal inlay can have the following dimensions: central thickness of between 15 µm to 30 µm; edge thickness of between 10 µm to 25 µm; anterior (or outermost) and posterior (or innermost) radii of curvature of between 6 mm to 8 mm; and diameter of between 2 mm to 2.5 mm. Such a corneal inlay can be implanted at a distance of between 100 µm to 150 µm from the anterior corneal surface. Such a corneal inlay reshapes the cornea wherein the corneal inlay is implanted, adding thickness to the central region thereof, which in turn increases its steepness and reduces its radius of curvature, resulting in increasing power.

An ophthalmic material as described herein may be obtained by a method comprising polymerizing the first monomer and the second monomer in the presence of SF and in the presence of a polymerization initiator.

Preferably the polymerization initiator selected from azobisisobutyronitrile (AIBN), ammonium persulfate (APS), tetramethyl ethylenediamine (TEMED), and benzoyl peroxide (BP), and more preferably the polymerization initiator is AIBN. Such initiators are known in the art and may initiate the polymerization reaction by, e.g., application of heat.

The initiator may be present in a proportion of 0.001-1 wt.% with respect to the total weight of the first and second monomers, in particular of 0.01-1wt.%.

In several embodiments polymerizing may comprise:
- mixing the first monomer with the second monomer, optionally in a suitable solvent, in particular in water, to provide a monomer mixture;
- adding the polymerization initiator into the monomer mixture to provide a polymerizing mixture;
- adding SF to the polymerizing mixture to provide a reaction mixture; and
- polymerizing the reaction mixture.

Mixing the first monomer with the second monomer to provide a monomer mixture, may be performed in a manner known in the art, e.g., in a reactor provided with mixing means such as a stirrer. In case a solvent is used, the solvent may be any suitable solvent such as water, ethanol and mixtures of ethanol and water (70/30 or 50/50), but may particularly be water. If the first and second monomers are in liquid form, there is no need to use any solvent. The first and second monomer may typically be added to the solvent in powder form.

Adding the polymerization initiator into the monomer mixture to provide a polymerizing mixture, may comprise mixing using ultrasound, e.g., by means of a sonicator.

Adding SF to the polymerizing mixture to provide a reaction mixture, has been found to provide ophthalmic materials of particularly good quality. Adding will typically be performed by mixing. SF may typically be previously dissolved in a suitable solvent, e.g., water, added in solution to the polymerizing mixture. The solution of SF to be added to the polymerizing mixture may be preferably be an aqueous solution of, e.g., 1 to 5wt.% of SF based on the total weight of water.

Suitable monomers, silk fibroin and proportions thereof, and particular or preferred embodiments thereof, as described above for the ophthalmic material apply to the method for preparation. Accordingly, the monomers, silk fibroin will be preferably mixed in the proportions described above.

In several embodiments polymerizing may be performed by applying heat to the reaction mixture, in particular by heating the reaction mixture to a temperature from 35 to 90 °C, in particular from 40 to 80 °C, more in particular from 50 to 75 °C. Suitable temperatures may be referred to as the polymerizing temperature. Heating may be performed in a manner known in the art, e.g., by placing the reaction mixture in an oven at the appropriate temperature or in a reactor provided with heating means.

Polymerizing may be performed by maintaining the reaction mixture at the polymerizing temperature for a suitable length of time, e.g., until the polymerization has been completed. Completion of the polymerization may be determined by, e.g., absence of water in a mould, which mould is used to cast the material. Polymerizing the reaction mixture may be performed with a lid of the mould on the mould to cover the reaction mixture, thereby improving the polymerizing, due to slow water evaporation and to less oxygen being present. Typically, polymerizing may be performed for 1-48 h, in particular for 3-36 h, more in particular for 5-24 h and yet more in particular for 8-12 h.

Once the polymerization is deemed complete, the resulting material is obtained in dry conditions.

The resulting ophthalmic material has a composition and properties as described above. In particular the ophthalmic materials will have the reacted first and second monomers and silk fibroin, and small amounts of initiator, e.g., in the percentage used in the preparation of the material, e.g., as described above. The ophthalmic material may also typically be obtained substantially free of solvents and in particular substantially free of water, i.e., will comprise less than 0.5 wt.% of solvent, e.g., water, and in particular, less than 0.1 wt.%.

The resulting ophthalmic material can itself have properties suitable for its direct application, e.g., to form a lens or an implant. In several embodiments the ophthalmic material, e.g., when the first and second monomers are selected from hydrophilic monomers, may be hydrated to form a hydrogel.

When the ophthalmic material is obtained from a first and second monomer selected from hydrophilic monomers, may also be referred to as a hydrophilic ophthalmic material or a hydratable ophthalmic material. A hydrogel may be obtained from the hydrophilic ophthalmic material by simply adding water to the ophthalmic material by means known in the art. As a mode of example, the ophthalmic material in solid form, for instance, in the form of sheets, may be soaked in water for 1 h, absorbing the water.

As indicated above, the ophthalmic material is well suited for ophthalmic lenses and/or implants. The ophthalmic material as such may be used for the formation of said ophthalmic lenses and/or implants. Such ophthalmic lenses and/or implants may already be used as such as ophthalmic lenses and/or implants.

When the ophthalmic material is obtained from a first and second monomer selected from hydrophilic monomers, which may also be referred to as hydrophilic ophthalmic material or hydratable ophthalmic material, ophthalmic lenses and/or implants may be obtained from the hydrophilic ophthalmic material by shaping it, prior to hydration or after hydration. Where the ophthalmic lenses and/or implants are obtained from the hydrophilic ophthalmic material prior to hydration, the ophthalmic lenses and/or implants will need to be hydrated prior to being used or implanted.

The ophthalmic material or the hydrogel (i.e., hydrated ophthalmic material) may be shaped to form ophthalmic lenses and/or implants by means known in the art and as specifically described also above.

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of ophthalmic materials, dimensions, components, configuration, etc.), within the general scope of the disclosure as defined in the claims.

The disclosure is further illustrated by the following examples without being limited thereto or thereby.

### EXAMPLE

### Preparation of Ophthalmic material

An ophthalmic material from 25 wt.% of HEMA and EGDMA monomers and 75wt.% of silk fibroin, based on the total weight of monomers and SF.

A monomers mixture was prepared by mixing HEMA and EGDMA in a proportion HEMA to EGDMA of 17:1, by stirring the monomers in liquid form.

A 0.6 wt.% of AlBN as polymerization initiator was added to with to the monomers mixture with respect to the total weight of HEMA and EGDMA.

The AIBN is dissolved into the monomer mixture by using ultrasound to provide a polymerization mixture.

Then, a 3% silk fibroin solution in water was prepared. The appropriate weight of polymerization mixture including AIBN was added to the silk fibroin solution in order to have a 25% in weight of monomers with respect to the total weight of monomers and SF. The silk fibroin solution was stirred with the polymerization mixture at 700 RPM for 2 minutes. The obtained reaction mixture was cast in a petri dish, covered with a lid and polymerized at 60° C overnight.

The same method was repeated using different proportions concentration of SF, monomers and polymerization initiator as detailed in the following table.

| Formulation | Wt.% of SF in water | Wt.% of monomer with respect to the SF solution in water | HEMA/EG DMA ratio | Wt.% AIBN with respect to monomer weight | % Monomer with respect to total monomer and SF |
|---|---|---|---|---|---|
| A | 3 | 1.98 | 17:1 | 0.6 | 40 |
| B | 6 | 1.98 | 17:1 | 0.6 | 24 |
| C | 3 | 0.66 | 17:1 | 0.6 | 18 |
| D | 6 | 0.66 | 17:1 | 0.6 | 10 |
| E | 3 | 0.75 | 3:1 | 0.6 | 20 |
| F | 3 | 0.75 | 10:1 | 0.6 | 20 |
| G | 3 | 0.75 | 30:1 | 1.2 | 20 |
| H | 3 | 0.75 | 17:1 | 0.6 | 20 |

### Properties of the Ophthalmic material

The ophthalmic material obtained was hydrated by adding water to the material and letting it soak for 1 hour to provide a hydrogel. Excess of water was removed.

The weight of the ophthalmic material obtained is increased resulting in a hydrogel with around 30 wt.% of water based on the total weight of hydrogel.

A hydrated ophthalmic material (hydrogel) was obtained that, in addition to being biocompatible, was transparent.

The properties of the materials A-H obtained were determined as follows.

The transparency (transmittance) was measured with a UV/Vis spectrophotometer, following a standard method. The hydrated material was placed in a cuvette with water and its transmittance is measured in the visible range, 400-800 nm. Five hydrogel samples were prepared, the samples being of the same size and shape, and having uniform thickness. Five samples were measured, and the transmittance was averaged in the 400-800 nm range. Transmittance of the hydrogel C was found to be of 98%, averaged from the 5 samples of the same material, which is a similar transparency to that of intraocular lenses.

The Young's Modulus (at 5% strain) of the hydrogels H was of 30-40 MPa, averaged from 5 samples of the same material. The method used was "tensile testing" as described above. Materials with such young modulus are stiffer (and thus less deformable) than the cornea (which typically has a young modulus of 1 MPa), and therefore are very suited for use as ophthalmic lens or implants.

Permeability measurements based on glucose diffusion measurements were performed for hydrogel H which showed a glucose diffusion coefficient of 10⁻⁷ - 10⁻⁸ cm²/s through the ophthalmic material as determined as described above.

The stability of hydrogel A was measured by immerging the hydrogel at 37° C in phosphate buffered saline (PBS) solution and was found to be stable for over 2 years. Further a more aggressive medium was used (0.05 U/mL protease XIV solution), and the material was found to be stable under these conditions for up to 3 hours. Hydrogel B was found to have a stability of 1 year under PBS and up to 1 month under 0.05 U/mL protease XIV solution. Hydrogels C, D and H were found to have a stability of 2 years under PBS and for up to 17 months in 0.05 U/mL protease XIV solution.

Therefore, the materials and corresponding hydrogels are suitable for a wide range of ophthalmic applications from contact lenses to implants.

The biocompatibility of the hydrogels A-H was determined as explained above.

The hydrogels H displayed 100% viability as determined by the MTT cytotoxicity test according to ISO 10993, both when using Human corneal fibroblasts (HCFs) or human cornea epithelial cells (HCE) immortalized by modification with the plasmid SV-40.

Thus, the prepared hydrogels were found to be highly biocompatible.

The hydrogels H displayed reduced cell adhesion as measured using HCFs, with direct seeding on the hydrogel and using phase contrast optical microscopy and Presto blue assay (PB), as described above. The assay was performed with reagent removal, and replacement with fresh medium and monitoring of cell proliferation, at 1, 5 and 7 days.

Thus, the prepared hydrogels were found to be non-toxic. Further they were also found to prevent conversion of fibroblasts into myoblasts, which is a marker of possible opacification, when compared to a control, e.g., plastic material for cell culture.

### Preparation of a corneal inlay

In an example of disclosure, a corneal inlay is designed based on the measured mechanical properties of the ophthalmic material that produces a three-dimensional modification in the central corneal curvature, as predicted by a Finite-Element Model simulation.

The corneal inlay is conceived as a positive meniscus, in the present example having low or zero optical power. The dimensions of the corneal inlay are: central thickness of 30 µm; edge thickness of 10 µm; anterior (or outermost) and posterior (or innermost) radii of 7 mm; and diameter of 2 mm. The corneal inlay is to be implanted at a distance of 100 µm from the anterior corneal surface.

## Claims

1. An ophthalmic material obtainable by polymerizing a first monomer and a second monomer in the presence of silk fibroin (SF) wherein the first monomer and the second monomer are different and are independently selected from a methacrylate, an acrylate, an acrylamide, a siloxane, a carbamate, a glycol, a vinyl and an allyl, and wherein the weight ratio of the first monomer to the second monomer is greater than 1:1.

2. The ophthalmic material of claim 1, wherein the weight proportion of the combination of the first and second monomers with respect to SF is of 10-40 wt.% to 60-90 wt.% based on the total weight of first monomer, second monomer and SF.

3. The ophthalmic material of claims 1 or 2, wherein the weight ratio of the first monomer to the second monomer is from 2:1 to 40:1, in particular from 3:1 to 35:1, more in particular from 5:1 to 30:1, yet more in particular from 7:1 to 25:1.

4. The ophthalmic material of any one of claims 1 to 3 wherein the first monomer and the second monomer are independently selected from a methacrylate, preferably are selected from hydroxy ethyl methacrylate (HEMA), ethylene glycol dimethacrylate (EGDMA), methacrylic acid (MAA), methyl methacrylate (MMA), oligo (ethylene glycol) methyl ether methacrylate (OEGMA), glycerol methacrylate (GMA), isobutyl methacrylate (IBMA), allyl methacrylate (AMA), 3-[tris(trimethylsiloxy)silyl] propyl methacrylate (TRIS), polypropylene glycol dimethylacrylate (PPGDMA) and methacryloyl oxyethyl phosphorylcholine (MPC); more preferably are selected from HEMA and EGDMA; and yet more preferably the first monomer is HEMA and/or the second monomer is EGDMA.

5. The ophthalmic material of any one of claims 1 to 4 wherein the first monomer and the second monomer are independently selected from hydrophilic monomers, in particular are selected from HEMA, EGDMA, EGPEA, DEG, and MPC, more in particular from HEMA and EGDMA, yet more in particular the first monomer is HEMA and/or the second monomer is EGDMA.

6. A hydrogel comprising the ophthalmic material of claim 5 and water, in particular comprising 10 to 40 wt.% of water, in particular from 12 to 30 wt.%, based on the total weight of the hydrogel.

7. The hydrogel of claim 6, having a Young's modulus from 20 to 70 MPa, in particular from 30 to 60 MPa.

8. The hydrogel of any one of claims 6 to 7, the ophthalmic material having a glucose permeability of 10⁻⁶ to 10⁻⁸ cm²/s.

9. An ophthalmic lens or implant comprising the ophthalmic material of any one of claims 1 to 5 or the hydrogel of any one of claims 6 to 8.

10. The ophthalmic lens or implant of claim 9, selected from a corneal inlay, a phakic lens, an intraocular lens, an aphakic intraocular lens, a corneal onlay, an intracorneal ring segment and a contact lens, preferably being a corneal inlay.

11. The ophthalmic lens or implant of claims 9 or 10, comprising the hydrogel and being a corneal inlay.

12. The ophthalmic lens or implant of claim 11 having an essentially circular shape and a diameter between 2-2.5 mm and/or having a central thickness between 15-30 µm.

13. The ophthalmic lens or implant of claims 11 or 12, the ophthalmic lens or implant having an anterior surface and a posterior surface having curvature radii of between 6 mm to 10 mm and, in particular, having curvature radii of between 6 mm to 8mm.

14. A method for preparing the material of any one of claims 1 to 5, comprising polymerizing the first monomer and the second monomer in the presence of SF in the presence of a polymerization initiator, preferably the polymerization initiator selected from azobisisobutyronitrile (AIBN), ammonium persulfate (APS), tetramethyl ethylenediamine (TEMED), and benzoyl peroxide (BP), and more preferably the polymerization initiator is AIBN.

15. The method of claim 14, wherein polymerizing comprises:
- mixing the first monomer with the second monomer, optionally in a suitable solvent, in particular in water, to provide a monomer mixture;
- adding the polymerization initiator into the monomer mixture, to provide a polymerizing mixture;
- adding SF to the polymerizing mixture to provide a reaction mixture; and
- polymerizing the reaction mixture.

16. The method of claims 14 or 15, wherein polymerizing is performed by applying heat to the reaction mixture, in particular by heating the reaction mixture to a temperature from 35 to 90 °C, in particular from 40 to 80 °C, more in particular from 50 to 75 °C.
